(19) **Europäisches Patentamt**
**European Patent Office**
**Office européen des brevets**

(11) **EP 4 191 604 A1**

(12) **EUROPEAN PATENT APPLICATION**

(43) Date of publication:
**07.06.2023 Bulletin 2023/23**

(21) Application number: **22158731.4**

(22) Date of filing: **25.02.2022**

(51) International Patent Classification (IPC):
**G16H 40/20** (2018.01)     **G06N 3/00** (2023.01)
**G06N 7/00** (2023.01)     **G06Q 10/00** (2023.01)

(52) Cooperative Patent Classification (CPC):
**G16H 40/20; G06N 3/00; G06N 7/00;** G16H 20/40

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.12.2021 US 202163285506 P**

(71) Applicant: **Koninklijke Philips N.V.**
**5656 AG Eindhoven (NL)**

(72) Inventors:
• **KOKER, Ekin**
**Eindhoven (NL)**

• **TELLIS, TELLIS**
**Eindhoven (NL)**
• **CHADUVULA, Siva Chaitanya**
**Eindhoven (NL)**
• **STAROBINETS, Olga**
**Eindhoven (NL)**
• **DALAL, Sandeep Madhukar**
**Eindhoven (NL)**
• **LI, Qianxi**
**Eindhoven (NL)**

(74) Representative: **Philips Intellectual Property & Standards**
**High Tech Campus 52**
**5656 AG Eindhoven (NL)**

(54) **SYSTEM AND METHOD FOR MEASURING AND OPTIMIZING TECHNOLOGIST AND/OR RADIOLOGIST CROSS-MODALITY PERFORMANCE IN IMAGING ACQUISITION AND READING WORKFLOWS**

(57) A non-transitory computer readable medium (26, 30) stores instructions executable by at least one electronic processor (20, 34) to perform a method (100) of distributing medical examinations to medical professionals. The method includes tracking examinations performed or reviewed by a plurality of medical professionals; calculating one or more metrics (38, 40) for each medical professional of the plurality of medical professionals based on the tracked examinations, the one or more metrics including a diversity metric indicative of a diversity of different types of examinations performed or reviewed by the medical professional; and allocating upcoming examinations among the medical professionals of the plurality of medical professionals based at least in part on the one or more metrics calculated for the medical professionals of the plurality of medical professionals.

**Figure 1**

EP 4 191 604 A1

**Description**

FIELD OF THE INVENTION

The following relates generally to the medical imaging arts, medical imaging device maintenance arts, medical imaging device examination procedure arts, medical professional scheduling arts, and related arts.

BACKGROUND OF THE IVENTION

Hospitals with radiology departments and imaging centers often provide services including imaging using a number of different medical imaging modalities, such as magnetic resonance imaging (MRI), computed tomography (CT), positron emission tomography (PET), single photon emission computed tomography (SPECT), fluoroscopic imaging, and so forth. Any given department or imaging center may provide some subset of these and/or other imaging modalities. In a common workflow, an imaging technologist operates the MRI, CT, PET, or other imaging device to acquire images of a patient in accordance with a radiology order issued by a physician treating the patient. The resulting radiology images are then read by a radiologist, who is a medical doctor with specialized training in radiology. Imaging technologists are valuable specialists, and radiologists are highly trained in their specialty as well. As such, these are valuable employees. To maximize the value of the radiology department or center, it is desirable to maintain a high throughput - consequently, technologists and radiologists are expected to perform at a high rate of efficiency. Radiologists, for example, are expected to log a certain number of relative value units (RVUs) in each work shift, where RVU is a metric that measures the number of examinations read by the radiologist weighted by the difficulty of each examination reading. To further enhance value of these medical professionals, it is generally advantageous for an imaging technician to be trained in multiple imaging modalities (e.g., MRI, CT, PET, ...) and likewise for a radiologist to be qualified to read radiology examinations of multiple imaging modalities. The high workload and difficult technical subject matter can lead to high turnover among technologist and radiologist staffs, especially among junior staff members with limited experience.

Hospitals with radiology departments and imaging centers can provide virtual over-the-shoulder support for less experienced technologists by enabling communication between a local tech and a remote expert user (i.e., a "super tech"). An expert user can virtually "step into" the imaging room via cameras and see the local tech's console via screen sharing functionality and can talk to the local tech via audio/video to guide them through an imaging exam. This type of assistance can help to reduce staff burnout and consequent turnover.

The following discloses certain improvements to overcome these problems and others.

SUMMARY OF THE INVENTION

In some embodiments disclosed herein, a non-transitory computer readable medium stores instructions executable by at least one electronic processor to perform a method of distributing medical examinations to medical professionals. The method includes tracking examinations performed or reviewed by a plurality of medical professionals; calculating one or more metrics for each medical professional of the plurality of medical professionals based on the tracked examinations, the one or more metrics including a diversity metric indicative of a diversity of different types of examinations performed or reviewed by the medical professional; and allocating upcoming examinations among the medical professionals of the plurality of medical professionals based at least in part on the one or more metrics calculated for the medical professionals of the plurality of medical professionals.

In some embodiments disclosed herein, a method of distributing medical examinations to medical professionals includes tracking examinations performed or reviewed by a plurality of medical professionals wherein the tracking includes tracking types of the examinations; calculating a diversity metric for each medical professional of the plurality of medical professionals based on the tracked types of examinations; and allocating upcoming examinations among the medical professionals of the plurality of medical professionals based at least in part on the diversity metrics calculated for the medical professionals of the plurality of medical professionals.

In some embodiments disclosed herein, a method of distributing medical examinations to medical professionals includes tracking examinations performed or reviewed by a plurality of medical professionals wherein the tracking includes tracking types of the examinations; calculating an efficiency metric for each medical professional of the plurality of medical professionals based on the tracked completion times for performing or reviewing the examinations against corresponding expected completion times for performing or reviewing the examinations; and allocating upcoming examinations among the medical professionals of the plurality of medical professionals based at least in part on the diversity metrics calculated for the medical professionals of the plurality of medical professionals.

One advantage resides in allocating examinations to medical professionals based on a diversity metric and/or an efficiency metric.

Another advantage resides in reducing boredom amongst medical professionals who work on only one type of examination.

Another advantage resides in reduced staff turnover due to working on only one type of examination.

Another advantage resides in reducing disruptions in the schedules of medical professionals.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

Fig. 1 diagrammatically illustrates a medical examination distribution apparatus in accordance with the present disclosure.

Fig. 2 diagrammatically illustrates a medical examination distribution method using the apparatus of Fig. 1.

DETAILED DESCRIPTION OF EMBODIMENTS

As noted previously, staff burnout and high turnover among imaging technologists and radiologists can occur due to high workloads and the technical difficulty of medical imaging and radiology reading tasks. However, it is recognized herein that another cause of staff burnout in radiology is boredom that could arise from working on similar types of studies or same modalities every day, and not having the chance to improve their expertise across different types of studies or modalities. For example, a radiologist who is always assigned digital radiograph (DR) examination readings, which are considered to be relatively simple, may thereby be deprived of the ability to improve expertise in more complex types of reading tasks such as MRI or CT readings. This can lead to boredom, as well as discontentment at not having the opportunity to expand into more advanced areas.

This problem of lack of diversity in work can also be self-motivated. For example, a radiologist who develops extensive expertise in reading one imaging modality develops high efficiency at that modality, and can quickly accumulate RVUs by performing many readings in that modality. In this setting, the radiologist may be reluctant to perform readings in other modalities as doing so will be more difficult and will result in a lower RVU accumulation rate.

The following relates to a system to distribute radiology examinations or readings to technologists or radiologists, respectively (hereinafter, imaging professionals), in a manner that optimally builds the expertise of the imaging professional.

There is a tendency for an imaging professional to specialize in a particular modality (e.g. computed tomography (CT)) or even sub-modality (e.g. brain CT), to the exclusion of other modalities/sub-modalities. While to some degree this is good as the professional becomes an expert in that modality/sub-modality, it is recognized herein that overspecialization also can leave the professional underdeveloped in other modalities.

In view of this, disclosed systems and methods provide for more diverse assignment of imaging tasks among technologists or radiologists, while still maintaining beneficial specialization skills. In some embodiments, the assignments of imaging tasks are based on analysis of two pieces of information for each examination handled by an imaging professional which are tracked by the disclosed systems and methods: (1) the modality (or even sub-modality); and (2) an efficiency metric, such as a comparison of the time to read an exam versus the expected time based on the relative value unit (RVU) value of that exam.

This information is used to calculate various metrics. A cross-modality score is calculated to measure how well balanced the professional is in terms of working across different modalities (or sub-modalities). The efficiency of the professional in each modality is also tracked. These values can be tracked over time.

In some embodiments, the system further includes an automated exam assignment module that takes the foregoing information into account in allocating examinations to imaging professionals. The general goal is to expand the expertise of the professional while continuing to assign enough cases in the professional's main areas of expertise to keep up his or her efficiency in those areas. This module can be implemented using real-time reinforcement learning and/or modeling, with the efficiency metrics of ongoing exams being used as the feedback for determining whether the imaging professional's expertise is increasing or decreasing in any given modality. If the expertise in one modality is decreasing then more cases of that modality may be assigned; whereas, if the expertise is holding steady or even increasing then some workload may be moved into other modalities.

While described herein with reference to distribution of assignments to imaging technicians and/or radiologists, more generally the disclosed techniques can be utilized for distribution of medical assignments to medical professionals who are qualified to perform a range of different kinds of tasks (e.g. different modalities in the illustrative example). For example, the approach could be used to distribute surgery assignments for different types of surgeries amongst a staff of surgeons.

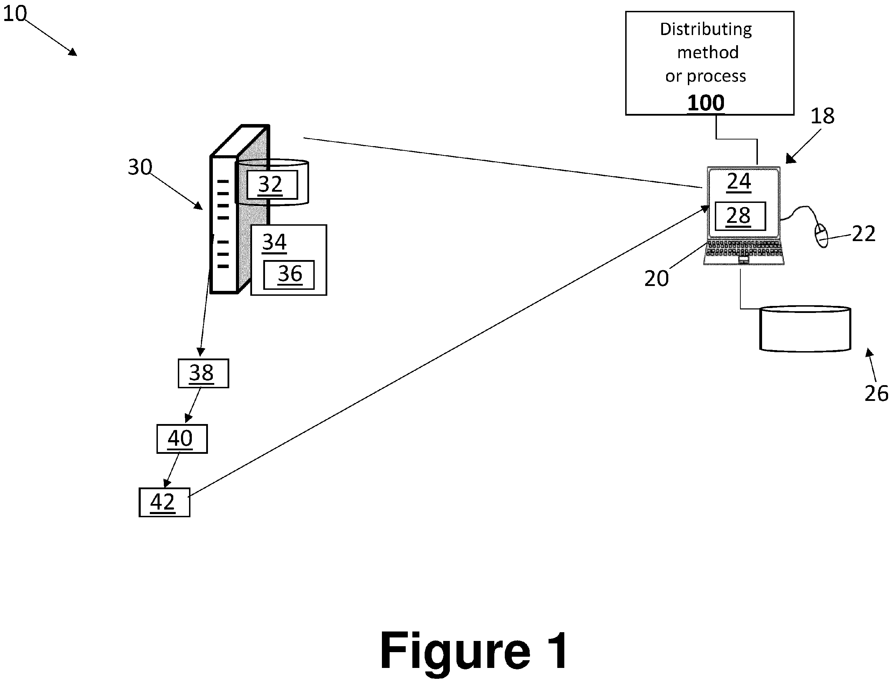

With reference to Fig. 1, an illustrative apparatus 10 for distributing medical examinations to one or more medical professionals. The apparatus 10 can comprise an electronic processing device 18 operable by the medical professionals. The electronic processing device 18 can comprise any suitable electronic device, such as a workstation computer, or more generally a computer, is operable by a user, such as a radiology department manager or administrator or the like. The electronic processing device 18 may also include a server computer or a plurality of server computers, e.g., interconnected to form a server cluster, cloud computing resource, or so forth, to perform more complex computational tasks. The electronic processing device 18 includes typical components, such as an electronic processor 20 (e.g., a microprocessor), at least one user input device (e.g., a mouse, a keyboard, a trackball, and/or the like) 22, and a display device 24 (e.g., an LCD display, plasma display, cathode ray tube display, and/or so forth). In some embodiments, the display device 24 can be a separate component from the electronic processing device 18, or may include two or more display devices.

The electronic processor 20 is operatively connected with one or more non-transitory storage media 26. The non-transitory storage media 26 may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation 18, various combinations thereof, or so forth. It is to be understood that any reference to a non-transitory medium or media 26 herein is to be broadly construed as encompassing a single medium or multiple media of the same or different types. Likewise, the electronic processor 20 may be embodied as a single electronic processor or as two or more electronic processors. The non-transitory storage media 26 stores instructions executable by the at least one electronic processor 20. The instructions include instructions to generate a visualization of a graphical user interface (GUI) 28 for display on the display device 24.

The electronic processing device 18 is also in communication with a server computer 30 that includes a database 32 (shown in Fig. 1 as a server computer) that stores records of examinations performed or reviewed by a plurality of medical professionals. The database 32 can store, for example, data related to which medical professional is assigned to perform which imaging and reading task assignments and how long these tasks take. The performance of staff members can be calculated using the volume of tasks and by the completion time of each task to generate key performance indicators (KPIs). These KPIs can be easily measured by recording them into one or more tables.

The server computer 30 can also include an electronic processor 34 configured to allocate upcoming examinations among the medical professionals based in part on the stored records of examinations performed or reviewed by the plurality of medical professionals. In some embodiments, the electronic processor 34 is configured to implement a reinforcement learning model (RL) 36.

The apparatus 10 is configured as described above to perform a method or process 100 of distributing medical examinations to medical professionals. The non-transitory storage medium 26 and/or the server computer 30 stores instructions which are readable and executable by the at least one electronic processor 20 of the electronic processing device 18 and/or the electronic processor 34 of the server computer 30 to perform disclosed operations including performing the servicing method or process 100. In some examples, the method 100 may be performed at least in part by cloud processing.

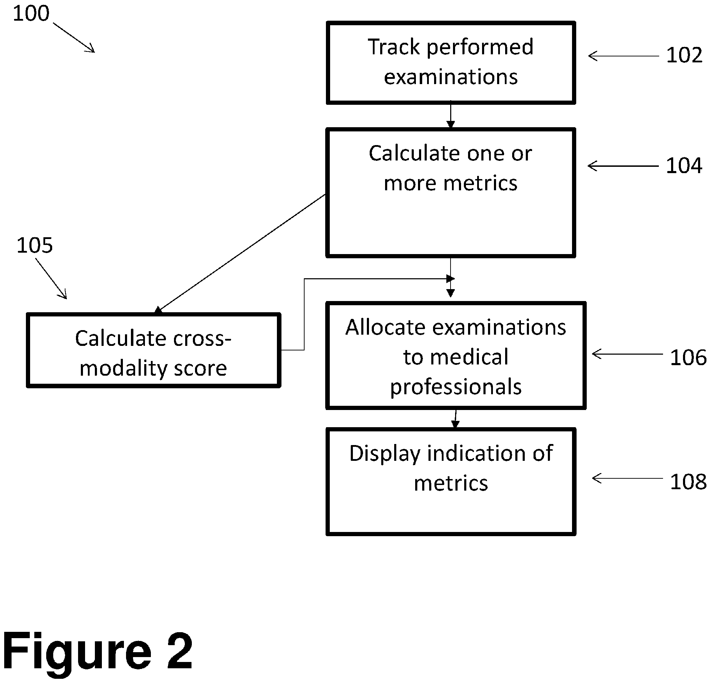

With reference to FIGURE 2, and with continuing reference to Fig. 1, an illustrative embodiment of an instance of the method 100 is diagrammatically shown as a flowchart. At an operation 102, examinations performed or reviewed by a plurality of medical professionals are tracked. The medical professionals can be, for example, radiologists, and the examinations can be, for example, radiology examination readings. In another example, the medical professionals can be imaging technologists and the examinations can be imaging examinations. For example, in a radiology context the tracking operation 102 can include, tracking a modality of a medical device (not shown) used in the imaging examinations (or imaging examination readings) performed by the plurality of medical professionals.

At an operation 104, one or more metrics for each medical professional of the plurality of medical professionals are calculated based on the tracked examinations. In some embodiments, the metric(s) can include a diversity metric 38 indicative of a diversity of different types of examinations performed or reviewed by the medical professional.

In other embodiments, the metric(s) can include an efficiency metric 40 calculated for each medical professional of the plurality of medical professionals. In one example, the efficiency metric 40 is indicative of a time taken by the medical professional to perform or review an examination against an expected time to perform the examination aggregated over the examinations performed or reviewed by the medical professional. In another example, the efficiency metric 40 is based on relative value unit (RVU) metrics assigned to the examinations performed or reviewed by the medical professional. For example, assuming that completing an imaging or reading examination more quickly is more efficient, the efficiency metric 40 can be calculated just by using the averages and standard deviations of task completion times. Radiology RVUs can also be used to measure how difficult an imaging or reading task is (e.g., a complicated brain scan using an MRI might be more difficult to read than a musculoskeletal X-Ray) to differentiate between different types of imaging tasks. RVUs can be used as weights to calculate a comprehensive efficiency score for a given radiologist or a technologist for a particular modality.

In some embodiments, at an (optional) operation 105, the diversity metric 38 can comprise a cross-modality score calculated based on the modality of the medical device or a type of examination and indicative of a diversity of examinations of different modalities performed or reviewed by the medical professional. Similarly, in another example, the efficiency metric 40 can comprise a cross-modality score calculated based on relative to a modality of the medical device used in the examination. These cross-modality scores can be calculated according to Equation 1:

$$CMS_s = 1 - \sum_{i=1}^{M} \left( \frac{v_{ms}}{v_s} \right)^2 \quad \forall s \qquad (1)$$

wherein s is a staff member index, $CMS_s$ is a cross-modality score for staff member s, the index $i$ is an index of modalities, M is a total number of modalities, $v_{ms}$ is a number of tasks completed for modality m by staff member $s$, and $v_s$ is a total number of tasks completed by staff member s. (In an alternative embodiment, $v_s$ and $v_{ms}$ may be RVU metrics).

In some examples, a sum of staff members' percentages for each modality is squared and subtracted from 1. In other examples, other data, such as study type, imaging scan protocol, and so forth can be provided with layered data (e.g., modality, study type, sub-group of study type (e.g., cardiac, brain, etc. studies, and so forth)) to generate the cross-modality score for each staff member. If the percentages are close to each other, their sum of squares will be reduced, and cross-modality score will be closer to 1. If the cross-modality score is close to 0, that staff member has a focus on a smaller number of modalities, which might indicate an expertise in those modalities. Below table is an example for a set of hypothetical radiologists with their reading percentage at each modality and the calculation of their cross-modality scores. In an example, a first radiologist (i.e., John Doe) has read equally from different modalities and therefore has a higher cross-modality score, whereas a second radiologist (i.e., Mary Brown) has only read from two modalities, majority from a single modality, and therefore has a lower cross-modality score (which could indicate expertise on the modalities that she regularly reads from).

For new staff members (e.g., radiologists and technologists), if they are new to the hospital or medical institution (but not entirely new), an initial cross-modality score can be calculated using their past records from previous institutions, if they consent to it. For entirely new staff members, their scores from formal training (certification exams, university transcripts, etc.) can be used as a proxy for cross-modality score until they establish a statistically significant set of task records. In addition to relative rating proposed here, an absolute rating based on industry standard reports (e.g. NRDR from American College of Radiology, American Board of Medical Specialties, American Board of Medicine, American National Standards Institute's Healthcare Informatics Standards Board (ANSI HISB), and so forth) is also of interest as it can help standardize scoring across institutions or hospitals and allow benchmarking. Cross-modality scores (CMS) can be even more granulized by breaking down each modality to its procedure types, i.e. a brain MR exam, a chest X-Ray exam, an abdomen, and pelvis CT exam, etc., as shown in Table 1 below.

Table 1

| Radiologist | MR | CT | US | XR | CMS |
|-------------|------|------|------|------|------|
| John Doe | 0.25 | 0.25 | 0.25 | 0.25 | 0.75 |
| Jane Doe | 0.3 | 0.4 | 0.2 | 0.1 | 0.7 |
| Jack Smith | 0.5 | 0.5 | 0 | 0 | 0.5 |
| Mary Brown | 0 | 0 | 0.8 | 0.2 | 0.32 |

As shown in Table 1, John Doe has the highest cross-modality core of 0.75, reflecting an even distribution of examination readings across the MR, CT, US, and XR modalities. Jane Doe has the highest score for CT examinations, but also has a reasonably diverse distribution across the other modalities, leading to a high cross-modality score of 0.7, Jack Smith has no experience in US examinations or X-ray examinations, as reflected in his low cross-modality score of 0.5, and Mary Brown has no experience in MR examinations or CT examinations and only limited experience in XR, and is heavily focused on US examinations, leading to her having the lowest cross-modality score of 0.32.

At an operation 106, upcoming examinations are allocated among the medical professionals of the plurality of medical professionals based at least in part on the one or more metrics calculated for the medical professionals of the plurality of medical professionals. For example, the upcoming examinations are distributed among the medical professionals of the plurality of medical professionals based on both the diversity metrics 38 and the efficiency metrics 40. In some embodiments the RL model 36 can be implemented to analyze the calculated diversity metrics 38 and the efficiency metrics 40. In another example, the RL model 36 can be implemented with training data, such as an upcoming or scheduled training session. The training data can establish proficiency, and upcoming training sessions can show a need for reinforcement of the RL model 36. In other embodiments, an individual medical professional can be assigned an examination having a modality in which the individual medical professional does not satisfy a predetermined modality metric. The examination can then be reassigned to a second individual medical professional having a modality in which the second medical professional does not satisfy a predetermined modality metric after initially assigning the examination to a first individual medical professional satisfying the predetermined modality metric.

To allocate the examinations, the assignments are searched to maximize an overall future score of the diversity metric 38 and/or the efficiency metric 40 for each medical professional. The complexity of the examinations is also balanced so that that one or two medical professionals on complex procedures are not bogged down by getting assigned all of these tasks. Another balancing mechanism to optimize is how many medical professionals are available per section and how busy they will be given the number of tasks expected during that section. In some examples, the allocation of the examinations can include considering schedules of the medical professionals, as well as a priority of the examinations (e.g., timing, expertise, and so forth).

At an operation 108, a representation 42 (e.g., a visual message) of the metric(s) 38, 40 can be output on the display device 24 of the electronic processing device 18.

The disclosed approaches for distributing medical examinations to medical professionals can also be usefully employed in the context of a remote operations command center (ROCC). This is a platform for hospitals with radiology departments and imaging centers to provide virtual over-the-shoulder support for less experienced technologists by enabling communication between a local tech and a remote expert user (super tech). An expert user can virtually "step into" the imaging room via cameras and see the local tech's console via screen sharing functionality and can talk to the local tech via audio/video to guide them through an imaging examination.

Technologists can also benefit by support from radiologists for issues such as checking the image quality before releasing the images to the Picture Archiving and Communication System (PACS) or other archival radiology examination storage system. The radiologist can also answer questions about particular protocols or sequences of the imaging exam, questions about the patient, etc. The ROCC provides an information technology (IT) infrastructure facilitating communication between radiologists and technologists, for example via a telephonic, video call, or other real-time interface and optionally also via asynchronous communication pathways such as an electronic ticketing system and chat functionality.

The risk that boredom may arise from working on similar types of studies or same modalities every day, and not having the chance to improve expertise across different types of studies or modalities, is also present in an ROCC setting. The disclosed cross-modality scoring for measuring radiologist and technologist cross-modality performance and thereby more effectively distributing tasks is thus applicable in the ROCC context. For example, the cross-modality scores of remote experts (super techs or radiologists, for example) can be used to more uniformly and equitably distribute assistance requests of local techs to ensure these experts benefit from a diversity of assistance requests. This can combat the tendency to refer assistance requests in a particular modality (for example) to an expert who heavily specializes in that modality, thus depriving that expert of a more diverse workload. Furthermore, it will be appreciated that within the ROCC context, the KPIs for computing the cross-modality scores can be readily obtained or derived from records maintained by the ROCC itself, e.g. the ROCC can log the assistance requests handled by each expert by modality or other breakdown(s).

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

**Claims**

1. A non-transitory computer readable medium (26, 30) storing instructions executable by at least one electronic processor (20, 34) to perform a method (100) of distributing medical examinations to medical professionals, the method including:

    tracking examinations performed or reviewed by a plurality of medical professionals;
    calculating one or more metrics (38, 40) for each medical professional of the plurality of medical professionals based on the tracked examinations, the one or more metrics including a diversity metric indicative of a diversity of different types of examinations performed or reviewed by the medical professional; and
    allocating upcoming examinations among the medical professionals of the plurality of medical professionals based at least in part on the one or more metrics calculated for the medical professionals of the plurality of medical professionals.

2. The non-transitory computer readable medium (26, 30) of claim 1, wherein the method (100) further comprises: outputting, on at least one display device (24), a representation (42) of the one or more metrics.

3. The non-transitory computer readable medium (26, 30) of either one of claims 1 and 2, wherein tracking examinations performed by a plurality of medical professionals includes:

   tracking a modality of a medical device used in the examinations performed by the plurality of medical professionals;
   wherein the diversity metric (38) comprises a cross-modality score calculated based on the modality of the medical device and indicative of a diversity of examinations of one of different modalities and a different examination type performed or reviewed by the medical professional.

4. The non-transitory computer readable medium (26, 30) of any one of claims 1-3, wherein calculating one or more metrics (38, 40) based on the tracked examinations includes:

   calculating an efficiency metric (40) for each medical professional of the plurality of medical professionals, the efficiency metric being indicative of a time taken by the medical professional to perform or review an examination against an expected time to perform the examination aggregated over the examinations performed or reviewed by the medical professional;
   wherein the upcoming examinations are distributed among the medical professionals of the plurality of medical professionals based on both the diversity metrics and the efficiency metrics.

5. The non-transitory computer readable medium (26, 30) of any one of claims 1-3, wherein calculating one or more metrics (38, 40) based on the tracked examinations includes:

   calculating an efficiency metric (40) for each medical professional of the plurality of medical professionals, the efficiency metric being based on relative value unit (RVU) metrics assigned to the examinations performed or reviewed by the medical professional;
   wherein the upcoming examinations are distributed among the medical professionals of the plurality of medical professionals based on both the diversity metrics and the efficiency metrics.

6. The non-transitory computer readable medium (26, 30) of claim 5, wherein calculating one or more metrics (38, 40) based on the tracked examinations includes:
   calculating a cross-modality score based on the efficiency metric (40) relative to one of a modality or examination type of a medical device used in the examination.

7. The non-transitory computer readable medium (26, 30) of any one of claims 1-6, wherein the allocating of examinations includes:
   applying a reinforcement learning model (RL) (36) to analyze the calculated one or more metrics (38, 40) to allocate the examinations to corresponding medical professionals.

8. The non-transitory computer readable medium (26, 30) of claim 7, wherein the RL model (36) is trained with training data comprising upcoming or scheduled training sessions.

9. The non-transitory computer readable medium (26, 30) of either one of claims 7 and 8, wherein the allocating of examinations includes:

   assigning an individual medical professional an examination having a modality in which the individual medical professional does not satisfy a predetermined modality metric; and
   re-assigning an examination to a second individual medical professional having a modality in which the second medical professional does not satisfy a predetermined modality metric after initially assigning the examination to a first individual medical professional satisfying the predetermined modality metric.

10. The non-transitory computer readable medium (26, 30) of any one of claims 1-9, wherein the allocating of examinations includes:
    allocating the examinations based on one or more of a schedule of the medical professionals and a priority of each examination.

11. The non-transitory computer readable medium (26, 30) of any one of claims 1-10, wherein the medical examinations comprise radiology examinations and the plurality of medical professionals comprise remote experts providing remote assistance to imaging technologists performing the radiology examinations via an information technology (IT) infrastructure, and the method (100) distributes the assistance requests to the remote experts (RE).

12. The non-transitory computer readable medium (26, 30) of claim 11, wherein the tracking utilizes log data of the IT infrastructure via which the remote experts (RE) provide remote assistance to the imaging technologists performing the radiology examinations.

13. A method (100) of distributing medical examinations to medical professionals, the method including:

   tracking examinations performed or reviewed by a plurality of medical professionals wherein the tracking includes tracking types of the examinations;
   calculating a diversity metric (38) for each medical professional of the plurality of medical professionals based on the tracked types of examinations; and
   allocating upcoming examinations among the medical professionals of the plurality of medical professionals based at least in part on the diversity metrics calculated for the medical professionals of the plurality of medical professionals.

14. The method of claim 13, wherein the examinations are radiology examinations, and the types of the examinations include examinations performed using different imaging modalities.

15. The method (100) of either one of claims 13 and 14, wherein the tracking further includes tracking completion times for performing or reviewing the examinations, the method further comprising:

   calculating an efficiency metric (40) for each medical professional of the plurality of medical professionals based on the tracked completion times for performing or reviewing the examinations against corresponding expected completion times for performing or reviewing the examinations;
   wherein the upcoming examinations are allocated among the medical professionals of the plurality of medical professionals further based on the efficiency metrics calculated for the medical professionals of the plurality of medical professionals.

Distributing
method
or process
**100**

18

24

28

22

20

26

30

32

34

36

38

40

42

EP 4 191 604 A1

# Figure 1

Figure 2

Europäisches
Patentamt

European
Patent Office

Office européen
des brevets

# EUROPEAN SEARCH REPORT

Application Number

EP 22 15 8731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/149193 A1 (JESTER ERIC [US] ET AL) 28 May 2015 (2015-05-28) * paragraphs [0060], [0080] – [0082], [0106] – [0108] * | 1-15 | INV. G16H40/20 G06N3/00 G06N7/00 G06Q10/00 |
| A | US 2013/262132 A1 (SANT ASHISH [CA] ET AL) 3 October 2013 (2013-10-03) * paragraph [0077] * | 1-15 | |
| A | US 2015/149192 A1 (JESTER ERIC [US] ET AL) 28 May 2015 (2015-05-28) * paragraph [0083] * | 1-15 | |

TECHNICAL FIELDS
SEARCHED        (IPC)

G16H
G06N
G06Q

The present search report has been drawn up for all claims

| Place of search | Date of completion of the search | Examiner |
|---|---|---|
| Munich | 20 July 2022 | Reinbold, Bernhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 15 8731

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2022

| Patent document cited in search report | | Publication date | Patent family member(s) | | Publication date |
|---|---|---|---|---|---|
| US 2015149193 | A1 | 28-05-2015 | US 2015149193 A1 | | 28-05-2015 |
| | | | US 2017091399 A1 | | 30-03-2017 |
| US 2013262132 | A1 | 03-10-2013 | NONE | | |
| US 2015149192 | A1 | 28-05-2015 | US 2015149192 A1 | | 28-05-2015 |
| | | | US 2017364643 A1 | | 21-12-2017 |

EPO FORM P0459

For more details about this annex : see Official Journal of the European Patent Office, No. 12/82